# EUROPEAN PATENT APPLICATION

(11) **EP 4 309 589 A1**
(43) Date of publication of application: **24.01.2024**
(21) Application number: 21931832.6
(22) Date of filing: 06.12.2021
(51) Int. Cl.: A61B 10/00, A61B 5/107, A61B 50/26

(54) **HEIGHT-ADJUSTABLE REMOTE SPECIMEN COLLECTION APPARATUS**

(30) Priority: 17.03.2021 KR 20210034672; 23.08.2021 KR 20210111177
(71) Applicant: Biot Korea Inc., Gwangju 61008 (KR)
(72) Inventor: CHANG, Yeong Jun, Seoul 06509 (KR); LEE, Jung Min, Seoul 06373 (KR)
(74) Representative: EP&C
(86) International application number: PCT/KR2021/018363
(87) International publication number: WO 2022/196891

(57) **Abstract**

A height-adjustable contactless specimen collection apparatus includes a body formed in a preset height and having a foot plate marked with foot positions for an examiner at a lower end thereof, a swab holder assembly disposed at an upper end portion of a front of the body and configured to hold a swab configured to collect a specimen, a lift disposed in the body and configured to move up and down the swab holder assembly, a face holder disposed in front of the swab holder assembly and configured to hold the face of the examinee, a swab collector disposed under the swab holder assembly and configured to collect the swab that has collected a specimen of the examinee, and a controller configured to control operation of the swab holder assembly, the lift, and the swab collector in response to remote control signals from an input device at a remote place.

## Description

### CROSS REFERENCE TO RELATED APPLICATION

The present application claims priority to Korean Patent Applications No. 10-2021-0034672, filed March 17, 2021, and Korean Patent Applications No. 10-2021-0111177, filed August 23, 2021, the entire contents of which are incorporated herein for all purposes by this reference.

### BACKGROUND

### Technical Field

The present disclosure relates to a specimen collection apparatus and, more specifically, to a height-adjustable contactless specimen collection apparatus that makes it possible to prevent secondary infection in the process of collecting a specimen because a specimen is collected in a contactless type due to operation by remote control and that makes it possible to smoothly collect a specimen from examinees with various body shapes because the height thereof is adjusted.

### Description of the Related Art

Recently, diseases due to virus infection are rapidly increasing.

In this case, since viruses easily transfer to other people through droplets, etc. from a patient, additional infection may widely occur, which may result in a pandemic.

Accordingly, it is required to quickly sort and isolate patients in the early stage of outbreak of viral diseases.

Accordingly, specimen collection is performed to sort viral disease patients.

That is, viral disease patients are sorted by inserting a swab into the nasal cavity of an examinee, collecting a specimen, putting and analyzing the specimen in various specimen analysis devices, and then determining whether the examinee has been infected with a viral disease.

However, contact unavoidably occurs between medical staff and an examinee in the process of collecting a specimen to determine whether the examinee has been infected with a viral disease, so there is a problem that additional infection may occur due to the contact.

For this reason, medical staff who are involved with specimen collection wear protective equipment including protective clothing.

However, wearing protective equipment is accompanied with interference with movement, so not only fatigue increases, but there is a problem that preparing protective equipment incurs considerably costs.

For these reasons described above, it has been being attempted to develop a specimen collection apparatus that can prevent secondary infection in the process of collecting a specimen by collecting a specimen in a contactless type in the corresponding field, but a satisfactory result is not accomplished up to now.

### (Related Art Documents)

(Patent Document 1) Korean Patent No. 10-2180927 (published 2020.11.19.)

### SUMMARY

The present disclosure has been made in an effort to solve the problems of the related art described above and an objective of the present disclosure is to provide a height-adjustable contactless specimen collection apparatus that makes it possible to prevent secondary infection in the process of collecting a specimen because a specimen is collected in a contactless type due to operation by remote control and that makes it possible to smoothly collect a specimen from examinees with various body shapes because the height thereof is adjusted.

In order to achieve the objectives, the present disclosure provides a height-adjustable contactless specimen collection apparatus that includes: a body formed in a preset height and having a foot plate marked with foot positions for an examiner at a lower end thereof; a swab holder assembly disposed at an upper end portion of a front of the body and configured to hold a swab configured to collect a specimen; a lift disposed in the body and configured to move up and down the swab holder assembly; a face holder disposed in front of the swab holder assembly and configured to hold the face of the examinee; a swab collector disposed under the swab holder assembly and configured to collect the swab that has collected a specimen of the examinee; and a controller disposed on any one surface of the body and configured to control operation of the swab holder assembly, the lift, and the swab collector in response to remote control signals that are input through an input device at a remote place.

According to the height-adjustable contactless specimen collection apparatus of the present disclosure, the swab holder assembly, the lift, and the swab collector are remotely controlled, so a specimen can be collected from an examinee in a contactless type, whereby it is possible to prevent secondary infection in the process of collecting a specimen and it is also possible to reduce costs and inconvenience due to wearing protective equipment of medical staff.

Further, according to the height-adjustable contactless specimen collection apparatus of the present disclosure, since the swab holder assembly is moved up and down, it is possible to smoothly collect a specimen from examinees with various body shapes by moving up and down the swab holder assembly, so the usability of the apparatus can be further increased.

Further, according to the height-adjustable contactless specimen collection apparatus of the present disclosure, since the swab holder assembly includes a camera, it is possible to collect a specimen while checking an image taken by the camera at a remote place, so swab insertion, etc. can be accurately performed and it is possible to increase accuracy and safety in specimen collection.

### BRIEF DESCRIPTION OF THE DRAWINGS

The above and other objectives, features and other advantages of the present disclosure will be more clearly understood from the following detailed description when taken in conjunction with the accompanying drawings, in which:
FIG. 1 is a perspective view showing the external appearance of a height-adjustable contactless specimen collection apparatus according to the present disclosure;

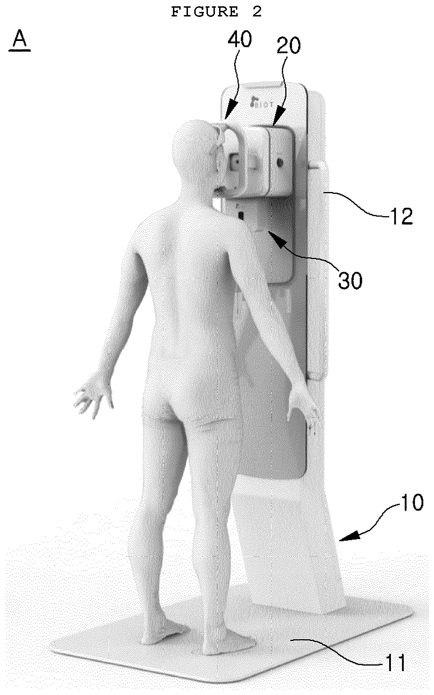

FIG. 2 is an exemplary view showing a use state of the height-adjustable contactless specimen collection apparatus according to the present disclosure;
FIG. 3 is a partial enlarged view showing a swab holder assembly of the height-adjustable contactless specimen collection apparatus according to the present disclosure;
FIG. 4 is an exemplary view showing supporting the face of an examinee through a face holder in the height-adjustable contactless specimen collection apparatus according to the present disclosure;
FIG. 5 is an exemplary view showing holding a swab through the swab holder assembly in the height-adjustable contactless specimen collection apparatus according to the present disclosure;
FIG. 6 is an exemplary view showing the state in which a swab held by the swab holder assembly is inserted in the nasal cavity of an examinee, in the height-adjustable contactless specimen collection apparatus according to the present disclosure;
FIG. 7 is an exemplary view showing the state in which the swab holder assembly has been maximally moved up by a lift in the height-adjustable contactless specimen collection apparatus according to the present disclosure;
FIG. 8 is an exemplary view showing the state in which the swab holder assembly has been maximally moved down by the lift in the height-adjustable contactless specimen collection apparatus according to the present disclosure;
FIG. 9 is an exemplary view showing the state in which a support of the lift has been maximally stretched in the height-adjustable contactless specimen collection apparatus according to the present disclosure;
FIG. 10 is an exemplary view showing the state in which the support of the lift has been maximally contracted in the height-adjustable contactless specimen collection apparatus according to the present disclosure;
FIG. 11 is an exemplary view showing collecting a swab through a swab collector in the height-adjustable contactless specimen collection apparatus according to the present disclosure;
FIG. 12 is an exemplary view showing the state before the swab collector is operated in the height-adjustable contactless specimen collection apparatus according to the present disclosure;
FIG. 13 is an exemplary view showing the state in which the swab collector starts to rotate in the height-adjustable contactless specimen collection apparatus according to the present disclosure;
FIG. 14 is an exemplary view showing the state in which the swab collector has further rotated in the height-adjustable contactless specimen collection apparatus according to the present disclosure;
FIG. 15 is an exemplary view showing the state in which the swab collector finished rotating in the height-adjustable contactless specimen collection apparatus according to the present disclosure;
FIG. 16 is an exemplary view showing fixing a swab collection container on the swab collector in the height-adjustable contactless specimen collection apparatus according to the present disclosure;
FIG. 17 is an exemplary view showing remote control by a controller in the height-adjustable contactless specimen collection apparatus according to the present disclosure;
FIG. 18 is an exemplary view showing a monitor display type in remote control in the height-adjustable contactless specimen collection apparatus according to the present disclosure;
FIG. 19 is an exemplary view showing another monitor display type in remote control in the height-adjustable contactless specimen collection apparatus according to the present disclosure; and

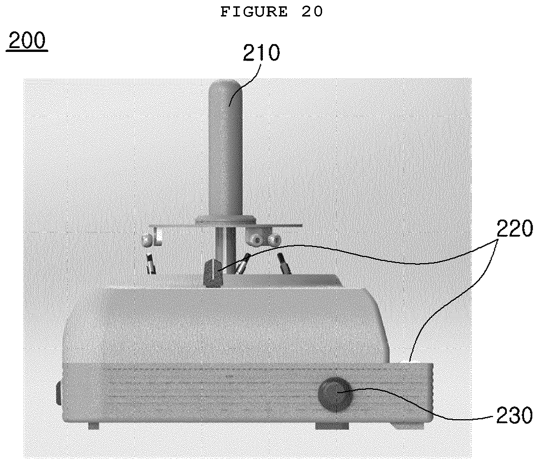

FIG. 20 is an exemplary view showing a type of an input device in the height-adjustable contactless specimen collection apparatus according to the present disclosure;

### DETAILED DESCRIPTION

Hereinafter, the present disclosure is described in detail on the basis of the accompanying drawings.

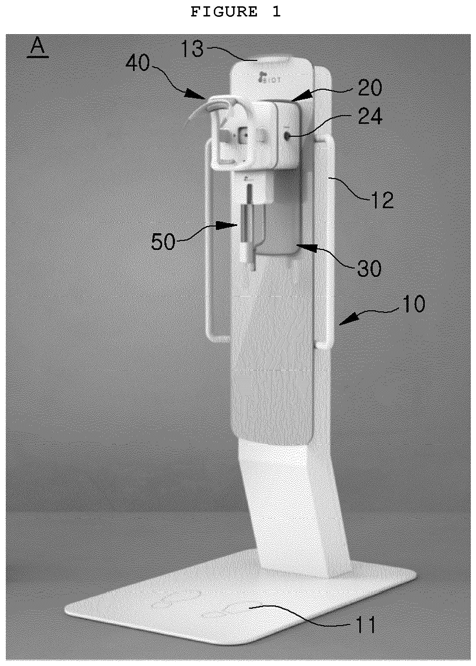

As shown in FIG. 1, a height-adjustable contactless specimen collection apparatus A according to the present disclosure includes a body 10, a swab holder assembly 20, a lift 30, a face holder 40, a swab collector 50, and a controller 60.

The body 10 of the present disclosure is formed in a preset height and has a foot plate marked with foot positions for an examinee at the lower end.

The body 10 has handles 12 protruding from both sides, so when an examinee holds the handles 12, the posture of the examinee can be stably maintained in the process of collecting a specimen.

A sensor (not shown in the drawings) that senses an examinee is disposed in the foot plate 11, so specimen collection can be performed only when it is determined that an examinee is on the foot plate in response to a signal from the sensor.

The sensor that senses an examinee is connected to a communication module of the controller 60 to be described below through a circuit, thereby being able to transmit a signal to a remote place.

The body 10 further has an indicator light 13, so the operation state of the swab holder assembly 20, the lift 30, and the swab collector 50 can be shown by light emission of the indicator 13.

The indicator 13 is positioned at the upper end of the front of the body 10, so it can be easily visually checked.

The indicator 13 emits different colors, depending on the operation states of the swab holder assembly 20, the lift 30, and the swab collector 50, so the operation states of the swab holder assembly 20, the lift 30, and the swab collector 50 can be discriminated by the emission colors.

The swab holder assembly 20 of the present disclosure is disposed at the upper end portion of the front of the body 10 and holds swabs 10 for collecting specimens.

The swab holder assembly 20 includes a holder 21 gripping and fixing a swab 100, so the swab 100 can be fixed by the holder 21.

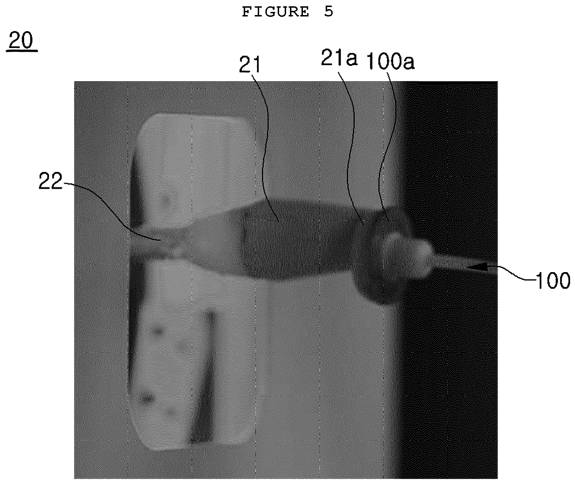

The holder 21, as shown in FIG. 5, includes a magnetic material 21a that generates magnetism, so the swab 100 can be simply fixed to the holder 21 by putting a magnetic material 100a disposed at the end of the swab 100 onto the magnetic material 21a of the holder 21.

The holder 21 further includes a sensor (not shown in the drawings) that senses pressure that is applied to the swab 100, so excessive insertion of the swab 100 can be prevented by stopping insertion of the swab 100 when the pressure sensed by the sensor in the process of inserting the swab 100 into the nasal cavity of an examinee exceeds preset maximum pressure.

The sensor that senses the pressure that is applied to the swab 100 is connected to the communication module through a circuit, thereby being able to transmit a signal to a remote place.

The swab holder assembly 20 includes a holding arm 22 that holds the holder 21 and has a plurality of joints to move and partially rotate in many directions, so as the holding arm 22 is moved and rotated, the holder 21 and the swab 100 fixed to the holder 21 can be moved and rotated. Accordingly, the swab 100 can be not only smoothly inserted into the nasal cavity of an examinee, but smoothly brought into contact with the inner surface of the nasal cavity.

The holding arm 22 can be formed in any common structure and type as long as it can smoothly move and partially rotate in many directions, so the holding arm 22 is not described in detail.

The swab holder assembly 20 includes a camera 23 that photographs the face of an examinee, so it is possible to accurately and safely collect a specimen through remote control by remotely controlling the apparatus while checking an image taken by the camera 23 through a monitor M in real time at a remote place.

The camera 23 is connected to the communication module to be described below through a circuit, thereby being able to transmit an image signal to a remote place.

The swab holder assembly 20 further includes an emergency switch 24, so when the emergency switch 24 is operated in an emergency situation, the operation of the holder 21 and the holding arm 22 can be initialized.

The lift 320 is disposed in the body 10 and moves up and down the swab holder assembly 20.

The lift 30 includes a support 31 that is contracted and stretched in the up-down direction by an actuator 51 (not shown in the drawings), so when the swab holder assembly 20 is coupled to the front end of the support 31 and the support 31 is contracted and stretched by the actuator 51, the swab holder assembly 20 can be moved up and down.

The actuator 51 can have any common structure and type as long as it can operate to contract and stretch the support 31, and a motor or a cylinder can be exemplified as the structure.

The lift 30 further includes a shutter (not shown in the drawings) disposed in front of the support 31, so when the support 31 is contracted and stretched and the swab holder assembly 20 is correspondingly moved up and down, exposure of the support 31 is prevented, whereby aesthetic deterioration due to exposure of the support 31 can be prevented.

Meanwhile, the lift 30 moves up and down the swab holder assembly 20 within the range of 0 ~ 800mm, whereby the swab holder assembly 20 can be positioned in front of the faces of examinees with various body shapes, and accordingly, it is possible to easily collect a specimen from not only common adults, but children.

The face holder 40 of the present disclosure is disposed in front of the swab holder assembly 20 and holds the face of an examinee.

The face holder 40 includes a contact frame 41 that comes into close contact with the forehead and the jaw of an examinee, so when the contact frame 41 comes into close contact with the forehead and the jaw of an examinee, movement of the examinee's face can be prevented while a specimen is collected.

The face holder 40 further includes an elastic member (not shown in the drawings) that elastically supports the contact frame 41, so the contact frame 41 can be brought more closely into contact with the forehead and the jaw of a user due to elastic supporting by the elastic member.

The swab collector 50 of the present disclosure is disposed under the swab holder assembly 20 and collects a swab 100 having a specimen taken from an examinee.

The swab collector 50 includes a rotary member 52 that is rotated forward and backward within a predetermined range of angle by the actuator 51, so when the rotary member 52 is rotated by the actuator 51, the rotary member 52 can be moved ahead of the swab holder assembly 20 from under the swab holder assembly 20.

The swab collector 50 further includes a retainer 53 that is disposed at the front end of the rotary member 52 and fixes a swab collection container 54 through the magnetism of a magnetic material 53a, so the swab collection container 54 can be fixed on the retainer 53 by putting a magnetic material 54a, which is disposed on a surface of the swab collection container 54, onto the magnetic material 53a of the fixing member 53.

The controller 60 of the present disclosure is disposed on any one surface of the body 10 and controls operation of the swab holder assembly 20, the lift 30, and the swab collector 50 in response to remote control signals that are input through an input device 200 at a remote place.

The controller 60 includes a communication module (not shown in the drawings) that communicates with a communication device (not shown in the drawings) at a remote place, so when remote control signals are input through the communication module, not only can the controller 60 control operation of the swab holder assembly 20, the lift 30, and the swab collector 50, but signals can be transmitted from the camera 23, the sensors, etc. to a remote place.

Meanwhile, the input device 200 includes: a lever 210 that generates a remote control signal relevant to operation of the swab holder assembly 20; a function switch 220 that generates remote control signals relevant to operation control of the lift 30 and the swab holder assembly 50; and an emergency switch 230 that generates remote control signals relevant to initialization of operation of the swab holder assembly 20, the lift 30, and the swab collector 50. Accordingly, when the lever 210, the function switch 220, and the emergency switch 230 operate and generate remote control signals, the operation of the swab holder assembly 20, the lift 30, and the swab collector 50 can be controlled.

Collecting a specimen through the height-adjustable contactless specimen collection apparatus A according to the present disclosure is described in detail hereafter.

First, an examinee takes a preset position.

Since the present disclosure includes the body 10 having the foot plate 11 at the lower end, an examinee entered an examination place can take a preset position by stepping onto the foot positions marked on the foot plate 11.

Since a sensor that senses an examinee is disposed in the foot plate 11 and a signal from the sensor is transmitted to a remote place, it is possible to recognize whether the examinee is in position by receiving a signal from the sensor at a remote place.

Next, a swab 100 for collecting a specimen is inserted into the nasal cavity and collects a specimen.

Since the swab holder assembly 20 disposed at the upper end portion of the front of the body 10 fixes a swab 100 by gripping the end of the swab 100 through the holder 21 in the present disclosure, it is possible to collect a specimen by inserting the front end of a swab 100 fixed by the holder 21 into the nasal cavity of an examinee.

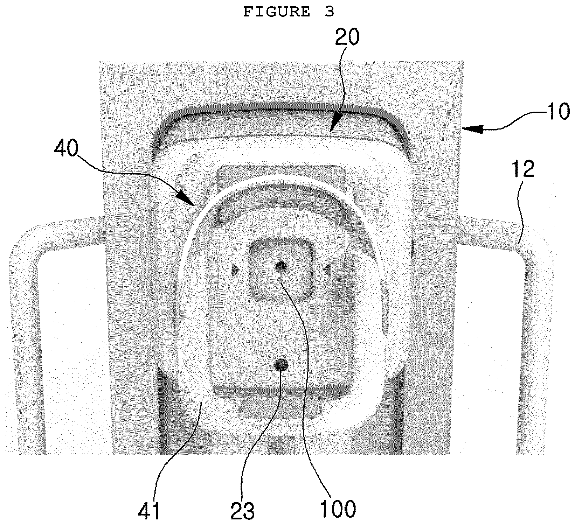

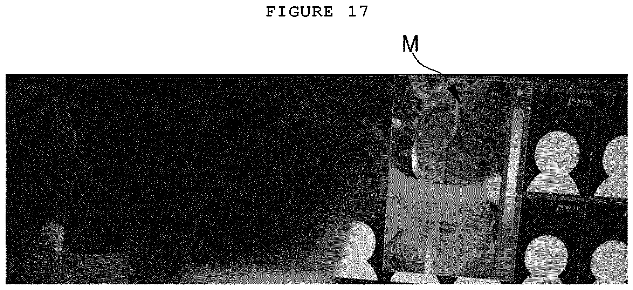

The swab holder assembly 20, as shown in FIG. 3, includes a camera 23 that photographs the face of an examinee, so when an image signal of the camera 23 is transmitted to and displayed on a monitor M at a remote place, as shown in FIG. 17, medical staff, that is, an examiner at the remote place can check in real time the face of the examinee in position.

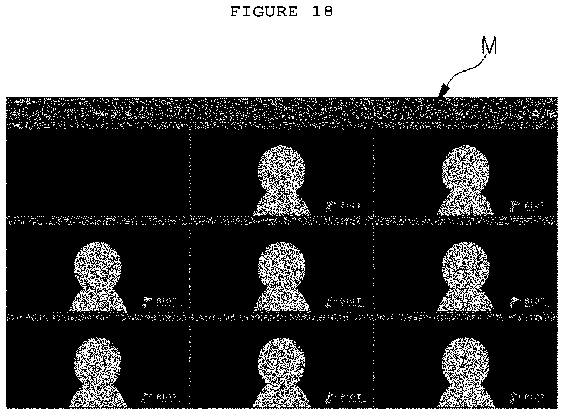

The monitor M can display images in separate sections, as shown in FIG. 18, so multi-control can be achieved through the controller 60.

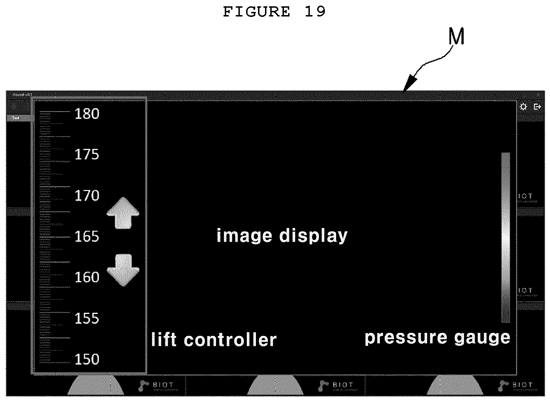

The monitor M not only can display an image of the face of an examinee, but, as shown in FIG. 19, can display the lifting width of the lift 30, pressure applied to a swab 100, etc., whereby remote control through the controller 60 can be easily achieved.

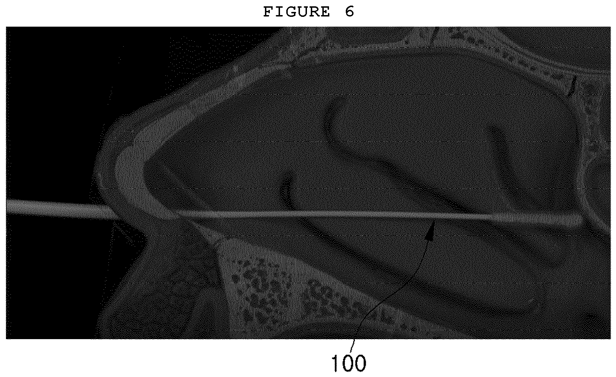

Meanwhile, since the holder 21 of the swab holder assembly 21 is held by the holding arm 22 having a plurality of joints and moving and partially rotating in many directions, it is possible to accurately insert a swab 100 into the nasal cavity of an examinee, as shown in FIG. 6, by moving the holding arm 22 in many directions, in more detail, forward and backward, left and right, and up and down through remote control that is performed while checking the face of the examinee in real time.

The holding arm 22 not only can be moved in many directions, but can be partially rotated, so a specimen can be smoothly collected in the nasal cavity of an examinee by partially rotating the front end of the holding arm 22 holding the holder 21 with a swab 100 inserted.

Since the holder 21 includes a sensor that senses pressure that is applied to a swab 100, it is possible to prevent injury to an examinee due to excessive insertion of a swab 100 by stopping inserting the swab 100 when pressure applied to the sensor due to close contact of the front end of the swab 100 with the inner surface of the nasal of the examinee exceeds preset maximum pressure.

The holder 21 and a swab 100 can be fixed by a medical staff before an examinee enters an examination place or an examinee himself/herself entered an examination place, so an examinee and a medical staff can be prevented from facing each other.

However, it may be difficult to insert a swab 100 into the nasal of an examinee, that is, it may be difficult to collect a specimen, depending on the body shapes, particularly, the heights of examinees.

That is, when an examinee is outstandingly taller or shorter than common adults, the swab holder assembly 20 cannot face the face of the examinee, so it is difficult to insert a swab 100 into the nasal cavity of the examinee and accordingly it may be difficult to collect a specimen.

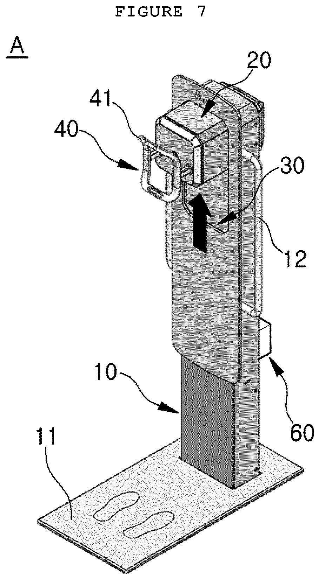

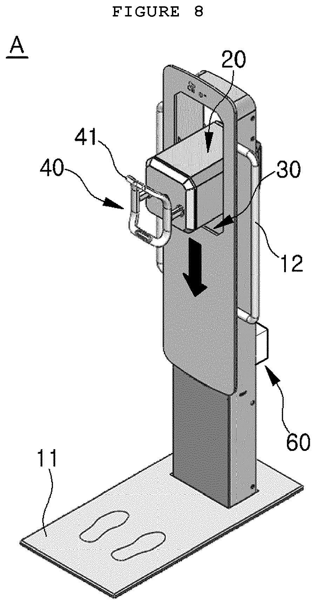

However, since the present disclosure includes the lift 30 that is disposed in the body and moves up and down the swab holder assembly 20, it is possible to move the swab holder assembly 20 accurately to the height of the face of an examinee by moving up and down the swab holder assembly 20 through the lift 30, as shown in FIG. 7 or 8. Accordingly, it is possible to easily insert a swab 100 into the nasal cavity of examinees regardless of the body shapes of the examinees, so it is possible to smoothly collect specimens.

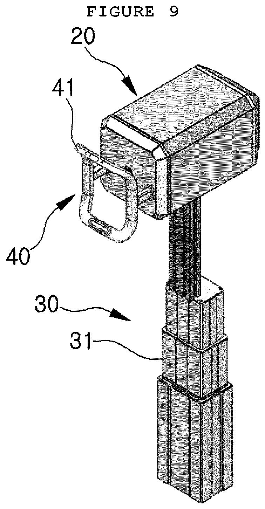

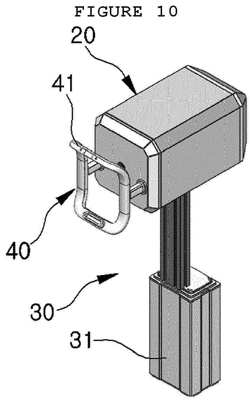

That is, since the lift 30 includes the support 31 that contracts or stretches in the up-down direction, it is possible to position the swab holder assembly 20 to face the face of an examinee by moving up and down the swab holder assembly 20 by contracting or stretching the support 31, as shown in FIG. 9 or 10, through remote control that is performed while checking the face of an examinee in real time. Therefore, it is possible to easily insert a swab 100 into the nasal cavity of examinees and accordingly it is possible to smoothly collect specimens.

Since the lift 30 moves up and down the swab holder assembly 20 particularly within the range of 0 ~ 800mm, it is possible to easily collect a specimen from children, etc. and normal adults as well.

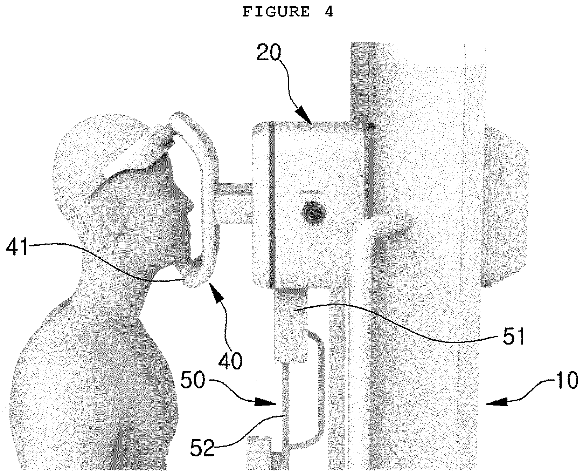

Meanwhile, since the face holder 40 is disposed in front of the swab holder assembly 20 in the present disclosure, movement of the face of an examinee can be prevented by bringing the examinee's face into close contact with the face holder 40 in the process of collecting a specimen, as shown in FIG. 4, so a specimen can be more smoothly collected.

Since the face holder 40 includes the contact frame 41 that comes into close contact with the forehead and the jaw of an examinee, when the contact frame 41 comes into close contact with the forehead and the jaw of an examinee, movement of the examinee's face can be prevented while a specimen is collected.

Since the face holder 40 further includes the elastic member (not shown in the drawings) that elastically supports the contact frame 41, the contact frame 41 can be brought more closely into contact with the forehead and the jaw of a user due to elastic supporting by the elastic member.

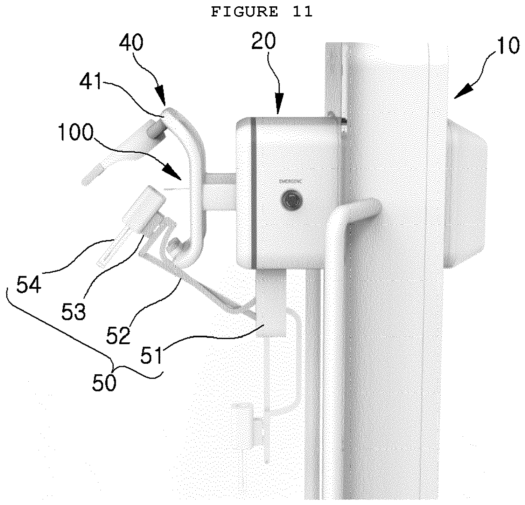

Next, a swab 100 that finishes collecting a specimen is collected, as shown in FIG. 11.

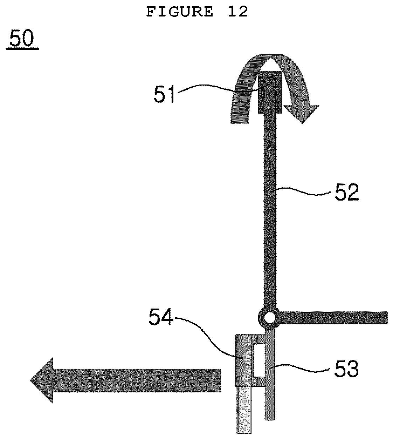

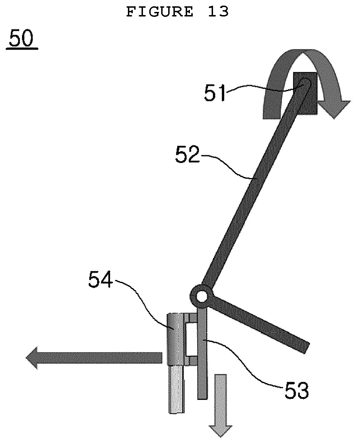

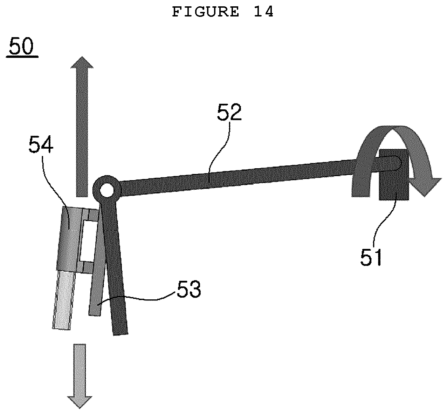

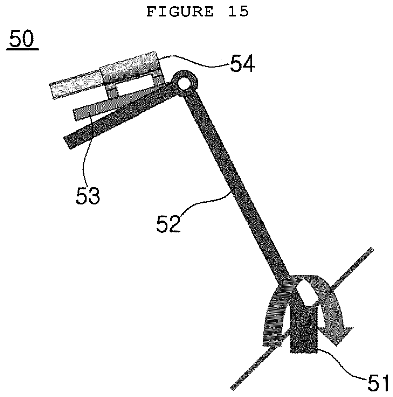

In the present disclosure, the swab collector 50 disposed under the swab holder assembly 20 includes: a rotary member 52 that is rotated forward and backward by the actuator 51; and a retainer 53 that is disposed at the front end of the rotary member 52 and fixes the swab collection container 54. Accordingly, the rotary member 52 is rotated toward the swab holder assembly 20, as shown in FIGS. 12 to 14, such that the inlet of the swab collection container 54 comes close to the front end of a swab 100 fixed on the holder 21 of the swab holder assembly 20, as shown in FIG. 15, through remote control that is performed while checking in real time the face of an examinee, that is, the front of the swab holder assembly 20, and in this state, the holding arm 22 is moved such that the holder 21 is moved forward and the front end of the swab 100 is inserted into the swab collection container 54, and then the rotary member 52 is rotated backward to return to the initial position, whereby the swab 100 that has collected a specimen can be collected.

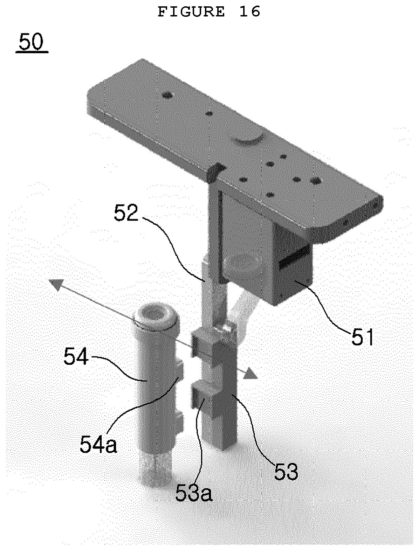

In this process, since the retainer 53, as shown in FIG. 16, fixes the swab collection container 54 using the magnetism generated by the magnetic material 53a, when an external force over the magnetism is applied, the swab collection container 54 can be simply separated from the retainer 53, so the swab 100 can be easily collected.

Meanwhile, the swab 100 collected by the swab collector 50 is put into an analysis device (not shown in the drawings) by a medical staff after the examinee go out of the examination place or is put into an analysis device by the examinee himself/herself who finishes specimen collection and then the specimen is analyzed in the analysis device, whereby whether the examinee has been infected can be determined.

Since the present disclosure described above is not limited to the embodiment described above, the present disclosure may be changed without departing from the spirit described in following claims and such change is included in the protection range of the present disclosure defined in the claims.

According to the present disclosure, it is possible to provide a height-adjustable contactless specimen collection apparatus that makes it possible to prevent secondary infection in the process of collecting a specimen and makes it possible to smoothly collect a specimen from examinees with various body shapes, so the present disclosure has industrial applicability.

## Claims

1. A height-adjustable contactless specimen collection apparatus comprising:
a body formed in a preset height and having a foot plate marked with foot positions for an examiner at a lower end thereof;
a swab holder assembly disposed at an upper end portion of a front of the body and configured to hold a swab configured to collect a specimen;
a lift disposed in the body and configured to move up and down the swab holder assembly;
a face holder disposed in front of the swab holder assembly and configured to hold the face of the examinee;
a swab collector disposed under the swab holder assembly and configured to collect the swab that has collected a specimen of the examinee; and
a controller disposed on any one surface of the body and configured to control operation of the swab holder assembly, the lift, and the swab collector in response to remote control signals that are input through an input device at a remote place.

2. The height-adjustable contactless specimen collection apparatus of claim 1, wherein the swab holder assembly includes:
a holder configured to fix the swab by gripping an end of the swab; and
a holding arm configured to hold the holder and having a plurality of joints to move and partially rotate in many directions.

3. The height-adjustable contactless specimen collection apparatus of claim 2, wherein the holder includes a sensor configured to sense pressure that is applied to the swab.

4. The height-adjustable contactless specimen collection apparatus of claim 2, wherein the swab holder assembly further includes a camera configured to photograph the face of the examinee.

5. The height-adjustable contactless specimen collection apparatus of claim 1, wherein the lift includes a support configured to contract or stretch in an up-down direction by an actuator.

6. The height-adjustable contactless specimen collection apparatus of claim 1, wherein the lift moves up and down the swab holder assembly within a range of 0 ~ 800mm.

7. The height-adjustable contactless specimen collection apparatus of claim 1, wherein the swab collector includes:
a rotary member configured to rotate forward and backward within a predetermined range of angle by an actuator; and
a retainer disposed at a front end of the rotary member and configured to fix the swab collector using magnetism of a magnetic material.

8. The height-adjustable contactless specimen collection apparatus of claim 1, wherein the controller includes a communication module configured to communicate with a communication device at a remote place.

9. The height-adjustable contactless specimen collection apparatus of claim 1, wherein the body includes an indicator configured to show an operation state of the swab holder assembly, the lift, and the swab collector by emitting light.

10. The height-adjustable contactless specimen collection apparatus of claim 2, wherein the swab holder assembly further includes an emergency switch configured to initialize operation of the holder and the holding arm.

11. The height-adjustable contactless specimen collection apparatus of claim 1, wherein the input device includes:
a lever configured to generate a remote control signal relevant to operation of the swab holder assembly;
a function switch configured to generate remote control signals relevant to operation control of the lift and the swab holder assembly; and
an emergency switch configured to generate remote control signals relevant to initialization of operation of the swab holder assembly, the lift, and the swab collector.
